# EUROPEAN PATENT APPLICATION

(11) **EP 4 417 705 A1**
(43) Date of publication of application: **21.08.2024**
(21) Application number: 22881402.6
(22) Date of filing: 14.10.2022
(51) Int. Cl.: C12Q 1/6883

(54) **USE OF MIR-625-3P AS BIOMARKER FOR DIAGNOSING SEVERITY OF PSORIASIS**

(30) Priority: 15.10.2021 KR 20210137391
(71) Applicant: Ajou University Industry-Academic Cooperation Foundation, Gyeonggi-do 16499 (KR)
(72) Inventor: LEE, Eun-So, Seoul 06291 (KR); PARK, Young Joon, Seoul 06310 (KR); KIM, Dong Chan, Suwon-si, Gyeonggi-do 16501 (KR); PARK, Ji Young, Suwon-si, Gyeonggi-do 16501 (KR); LEE, Soo-Jin, Suwon-si, Gyeonggi-do 16701 (KR)
(74) Representative: Hutter, Anton
(86) International application number: PCT/KR2022/015595
(87) International publication number: WO 2023/063775

(57) **Abstract**

The present invention relates to a use of miR-625-3p as a biomarker for diagnosing the severity of psoriasis, and more specifically, to: a composition or kit for diagnosing the severity of psoriasis, the composition or kit containing a material for detecting a miR-625-3p biomarker; and a method for diagnosing the severity of psoriasis by using the miR-625-3p as a biomarker. The biomarker for diagnosing psoriasis according to the present invention has the advantage of enabling the early objective diagnosis of psoriasis at a molecular level and the accurate determination of the severity of psoriasis, thus making it possible to increase the treatment efficiency of a chronic disease diagnosis.

## Description

### [Technical Field]

The present invention relates to the use of miR-625-3p as a biomarker for diagnosing the severity of psoriasis, and more specifically to a composition for diagnosing the severity of psoriasis or a kit for diagnosing the severity of psoriasis, including a material for detecting a miR-625-3p biomarker, and a method for diagnosing moderate-to-severe psoriasis using the miR-625-3p as a biomarker.

### [Background Art]

Psoriasis is a relatively common skin inflammatory disease, which occurs in 1 to 3% of the population, and it is a disease that requires long-term treatment by repeating improvement and exacerbation after onset at a young age. It has been reported that arthritis is induced in about 10% of patients with psoriasis, and it has also been reported to be associated with cardiovascular disease and metabolic syndrome.

There are the psoriasis area severity index (PASI) and body surface area (BSA) as biomarkers for evaluating the disease activity of psoriasis, but since these are judged by the clinician by looking at the condition of the skin, the subjectivity of the clinician may be involved in the judgment. Therefore, biomarkers that can objectively diagnose psoriasis are needed *(*Advances in Clinical and Experimental Medicine. 2017;26(5):851-6).

Exosomes are the smallest (30-120 nm) extracellular vesicles and include various biomolecules such as microRNA (miRNA), nucleic acids and proteins that enable cell-to-cell communication. The stable lipid bilayer of exosomes prevents the degradation of biomolecules. In addition, damaged keratinocytes can secrete exosomes, and psoriatic keratinocytes secrete exosomes due to excessive inflammation and abnormal differentiation *(*ACS Nano. 2020;14(10):12732-48; Cell Death & Disease 2022;13(1):1-13).

The miRNA is a small-sized non-coding RNA (20 to 25 nucleotides) known to play a role in regulating gene expression, and it has been reported that miRNA is involved in the proliferation and activation of T cells and is differentially expressed in psoriasis patients compared to healthy control groups *(*Biomedical Reports. 2018;9(5):367-74). Since circulating exosomal miRNAs are known to correlate with diseases with remarkable stability, they have been used as potential biomarkers reflecting disease activity for diseases such as cancer, hepatitis, dermatomyositis and neurodegenerative diseases.

In this regard, there are more than 250 differentially expressed miRNAs in the skin or blood of psoriasis patients, but since miRNAs are small single-stranded non-coding RNA molecules that are vulnerable to external stimulation and degradation, there are limitations in diagnosing psoriasis patients by using miRNAs.

However, the inventors of the present invention focused on the fact that miRNAs in circulating exosomes could be utilized for diagnosing psoriasis as a relatively stable biomarker and made diligent efforts to invent a novel, highly accurate biomarker that can objectively diagnose psoriasis using miRNA in circulating exosomes, and as a result, the present invention was completed by confirming that not only is the expression of miR-625-3p in circulating exosomes expressed at a significantly higher level in psoriasis, but also the expression level of miR-625-3p faithfully reflects the severity of the psoriasis disease.

### [Disclosure]

### [Technical Problem]

An object of the present invention is to provide a novel biomarker that is capable of accurately diagnosing the severity of psoriasis.

### [Technical Solution]

In order to achieve the above object, the present invention provides a composition for diagnosing the severity of psoriasis, including a material for detecting a miR-625-3p biomarker.

In the present invention, the material for detecting may include any one or more of a primer, a probe and an antisense nucleotide that specifically binds to the miR-625-3p biomarker.

In the present invention, the composition may further include a material for detecting a miR-4488 biomarker and/or a material for detecting a miR-342-3p biomarker.

In the present invention, the material for detecting may include any one or more of a primer, a probe and an antisense nucleotide that specifically binds to the miR-4488 biomarker and/or the miR-342-3p biomarker.

In addition, the present invention provides the use of a miR-625-3p biomarker for the diagnosis of the severity of psoriasis.

In addition, the present invention provides the use of a miR-625-3p in the preparation of a reagent for diagnosing the severity of psoriasis.

In addition, the present invention provides a method for diagnosing the severity of psoriasis, including the step of measuring the expression level of a miR-625-3p biomarker in a subject in need of the diagnosis of the severity of psoriasis severity.

In addition, the present invention provides a kit for diagnosing the severity of psoriasis, including the composition.

In addition, the present invention provides a method for providing information for diagnosing moderate-to-severe psoriasis, including the steps of:
(a) measuring the expression level of miR-625-3p in a sample isolated from a patient; and
(b) comparing the expression level of the miR-625-3p with the expression level of miR-625-3p in a mild psoriasis sample.

In the present invention, the expression level of miR-625-3p may be measured by using reverse transcriptase polymerase reaction, competitive reverse transcriptase polymerase reaction, real-time reverse transcriptase polymerase reaction, RNase protection assay or gene chip.

In the present invention, the sample may be a tissue, cell, blood or serum sample.

In the present invention, the sample may include exosomes that are isolated from tissue, cells, blood or serum.

In the present invention, the method for providing information may be characterized in that when the expression level of the miR-625-3p increases by 3.46 times or more compared to the expression level of miR-625-3p in a mild psoriasis sample, it is diagnosed as moderate-to-severe psoriasis.

In the present invention, step (a) may additionally measure the expression level of miR-4488 and/or miR-342-3p in the sample isolated from the patient, and step (b) includes the step of comparing the expression level of the miR-4488 with the expression level of miR-4488 in the mild psoriasis sample and/or comparing the expression level of the miR-342-3p with the expression level of miR-342-3p in the mild psoriasis sample.

In the present invention, the method for providing information may be characterized in that when the expression level of the miR-4488 increases by 2.375 times or more compared to the expression level of miR-4488 in the mild psoriasis sample and/or the expression level of the miR-342-3p increases by 1.657 times or more compared to the expression level of miR-342-3p in the mild psoriasis sample, it is diagnosed as moderate-to-severe psoriasis.

In the present invention, the expression level of the miR-4488 and/or miR-342-3p may be measured by using reverse transcriptase polymerase reaction, competitive reverse transcriptase polymerase reaction, real-time reverse transcriptase polymerase reaction, RNase protection assay or gene chip.

### [Advantageous Effects]

Since the biomarker for diagnosing psoriasis according to the present invention can objectively diagnose psoriasis at an early stage at the molecular level and accurately determine the severity of psoriasis disease, it has the advantage of increasing the treatment efficiency of diagnosing a chronic disease.

### [Description of Drawings ]

FIG. 1 is the results of collecting blood and skin tissue from psoriasis patients to extract exosomes, and then screening for differentially expressed miRNA showing significant differences using next-generation sequencing (NGS), and validating the expression pattern by selecting upwardly and downwardly expressed miRNA to perform quantitative real-time PCR thereon. The significance of correlations was tested by using Spearman's rank correlation test. *P<0.05, **P<0.01.
FIG. 1a is a mimetic diagram showing the experimental scheme of exosomal miRNA acquisition and analysis (screening phase shown in blue arrows (dotted lines) and validation phase shown in red arrows (solid lines).
FIG. 1b shows the differentially expressed genes (DEGs) on volcano plot. Selected exosomal miRNAs are shown in bold.
FIG. 1c shows miRNAs downregulated in the plasma of psoriasis patients with PASI >10.
FIG. 1d shows miRNAs upregulated in the plasma of psoriasis patients with PASI >10.
FIG. 1e shows the qRT-PCR results of upregulated miRNAs in skin of psoriasis patients.
FIG. 1f shows the skin miR-625-3p level plotted against PASI and BSA.
FIG. 2 is the results of verifying the diagnostic accuracy of elevatedly expressed miRNAs that showed significant differences in screening.
FIGS. 2a and 2b are the results of plotting plasma miR-625-3p, miR-4488 and miR-342-3p level against (A) PASI and (B) BSA which are psoriasis severity indices, respectively. The significance of the correlation was tested using the Spearman's rank correlation test.
FIG. 2c is the results of confirming the sensitivity and specificity through the ROC (receiver operating characteristic) curve and associated AUC value of each upregulated exosomal miRNAs.
FIG. 2d is a mimetic diagram showing the experimental scheme for analysis of exosomal miRNA levels before-and-after treatment depending on the active or inactive state of patients with Behçet's disease.
FIG. 2e is the results of confirming the difference in miRNA expressions by performing qRT-PCR after extracting exosomes from plasma depending on the active or inactive state of patients with Behcet's disease.
FIG. 2f is the results of extracting exosomes from the plasma of patients with Behcet's disease before and after treatment and performing qRT-PCR to confirm the difference in miRNA expressions between a group showing an improvement in PASI of 50 or more after treatment and a group not showing the same. *P < 0.05.
FIG. 3 is the results of confirming the source of exosomal miRNA through the in situ hybridization (ISH) technique in paraffin tissue.
FIGS. 3a and 3b are the results of detecting the expression of miR-625-3p in the skin detected using ISH in (a) a low power field (x100) and (b) a high power field (x400), respectively.
FIGS. 3c and 3d are the results that in order to determine whether the elevatedly expressed miRNAs were derived from keratinocytes or T cells, HaCaT cells and JurkaT cells were used to treat (c) collected cells and (d) supernatants with IL-12 (Th1), IL-12 (Th1) and IL-12 (Th1). 23 (Th17), and the expression levels of miR-625-3p and miR-4488 were determined through qRT-PCR. *P < 0.05 and **P < 0.01.
FIG. 4 is the results of confirming the possibility of association with the IGF-1 signaling system of miR-625-3p through the miRNA database. Data are presented as mean ± SEM. Horizontal lines above the bars represent statistical differences and statistical comparisons between categories. *P < 0.05, **P < 0.01, and ****P < 0.0001.
FIG. 4a is the results of predicting the target and function of miR-625-3p using three miRNA databases (miRDB/TargetScan/miRTarBase).
FIG. 4b is the results of confirming the expression level after transfection with a miR-625-3p mimic.
FIG. 4c is a qRT-PCR result of IGF-1 signaling genes after transfection with a miR-625-3p mimic.
FIG. 4d is the results of western blotting analysis of Akt protein in HaCat cells cultured for 48 hours after transfection with a miR-625-3p mimic.
FIG. 4e is the results confirming an increase in the expression level of the Ki67 gene, which increases during cell division after transfection with a miR-625-3p mimic.
FIG. 4f is the results of confirming the survival of HaCaT cells by CCK8 assay at different dopamine concentrations for 24, 48 and 72 hours after transfection with a miR-625-3p mimic.

### [Modes of the Invention]

Unless defined otherwise, all technical and scientific terms used in the present specification have the same meanings as commonly understood by one of ordinary skill in the art to which the present invention pertains. In general, the nomenclature used in the present application is that which is well-known and commonly used in the art.

All numbers expressing the sizes, amounts and physical properties of features used in the present specification and claims are to be understood as being modified in all instances by the term "about." Accordingly, unless indicated to the contrary, the numerical parameters disclosed in the specification and claims are approximations that can vary depending on the desired properties sought to be obtained by those skilled in the art by using the teachings disclosed in the present specification.

In the present invention, miR-625-3p, which can objectively diagnose psoriasis with miRNA in circulating exosomes, was identified as a novel biomarker for diagnosing the severity of psoriasis. In the diagnosis of psoriasis, the expression level of miR-625-3p showed a significant correlation according to the severity, and it was confirmed that miR-625-3p is a biomarker that reflects the severity of psoriasis disease.

Accordingly, in one aspect, the present invention relates to a composition for diagnosing the severity of psoriasis disease, including a material for detecting a miR-625-3p biomarker.

In the present invention, the material for detecting may include at least any one or more a primer, a probe and an antisense nucleotide that specifically binds to the miR-625-3p biomarker, but the present invention is not limited thereto.

In the present invention, the miR-625-3p biomarker may be characterized in that it is possible to determine the severity of psoriasis disease.

In the present invention, the composition may be characterized in that it further includes a material for detecting a miR-4488 biomarker and/or a material for detecting a miR-342-3p biomarker.

In the present invention, the material for detecting may include at least any one or more of a primer, a probe and an antisense nucleotide that specifically binds to the miR-4488 biomarker and/or the miR-342-3p biomarker, but the present invention is not limited thereto.

As used herein, the term "miR", "miRNA", "micro RNA" or "microRNA" refers to 21 to 23 non-coding RNAs that post-transcriptionally regulate gene expression by promoting the degradation of target RNAs or inhibiting the translation thereof. The mature sequences of the miRNAs used herein can be obtained from the miRNA database (http://www.mirbase.org).

In general, microRNA is transcribed as a stem-loop (primary miRNA or pri-miRNA) precursor with a hairpin structure called pre-miRNA and about 70-80 nt (nucleotides) in length. These pri-miRNAs may include several miRNA precursors, and are processed into precursor miRNAs (pre-miRNAs) having a hairpin structure by the action of enzymes. Subsequently, the pre-miRNA moves out of the nucleus, and the hairpin structure is cleaved by RNase enzyme (Dicer) in the cytoplasm. In this process, Dicer binds to the 3' end of the hairpin, cleaves a loop connecting the 3' and 5' arms, and forms an unstable double-stranded miRNA, and ultimately, mature miR-3p and miR-5p are formed

As described above, miR-625-3p according to the present application is derived from the 3p end of pre-miRNA miR-625 having a hairpin structure. In the present application, it was found that miR-625-3p was particularly elevated in psoriasis patients. The miR-625-3p biomarker according to the present application includes mature miR-625-3p as well as a precursor pre-miR-625 which is capable of producing the same.

In one aspect of the present invention, human-derived pre-miR-625 including miR-625 may be represented by SEQ ID NO: 1: AGGGUAGAGGGAUGAGGGGGAAAGUUCUAUAGUCCUGUAAUUA GAUCUCAGGACUAUAGAACUUUCCCCCUCAUCCCUCUGCCCU, and miR-625-3p may be represented by SEQ ID NO: 2: GACUAUAGAACUUUCCCCCUCA, and the underlined part in SEQ ID NO: 1 is a sequence forming a loop. Pre-miR-625 includes both of 5p and 3p sequences based on the loop, and when a probe and/or primer to be described below is used for detection, it is preferable to design to specifically recognize 3p.

In another aspect of the present invention, the human-derived pre-miR-4488 sequence including miR-4488 may be represented by SEQ ID NO: 3: GGUAGGGGGCGGGCUCCGGCGCUGGGACCCCACUAGGGUGGCGCCUUG GCCCCGCCCCGCCC, and miR-4488 may be represented by SEQ ID NO: 4: AGGGGGCGGGCUCCGGCG.

In still another aspect of the present invention, human-derived pre-miR-342 including miR-342 may be represented by SEQ ID NO: 5: GAAACUGGGCUCAAGGUGAGGGGUGCUAUCUGUGAUUGAGGGACAUG GUUAAUGGAAUUGUCUCACACAGAAAUCGCACCCGUCACCUUGGCCUA CUUA, and miR-342-3p may be represented by SEQ ID NO: 6: UCUCACACAGAAAUCGCACCCGU, and the underlined part in SEQ ID NO: 5 is a sequence forming a loop. Pre-miR-342 includes both of 5p and 3p sequences based on the loop, and when a probe and/or primer to be described below is used for detection, it is preferable to design to specifically recognize 3p.

As used herein, the term "biomarker" or diagnostic marker is a material that can diagnose by distinguishing a sample from a patient with psoriasis from a control group, and it includes non-coding nucleic acids that show an increased expression pattern in samples from patients with disease or patients with high severity of disease compared to control groups.

As used herein, the term "diagnosis" includes determining the susceptibility of a test subject to a specific disease or disorder, determining whether a test subject currently has a specific disease or disorder, determining the prognosis of a subject with a specific disease or disorder, and determining whether the disease recurs after treatment or therametrics (e.g., monitoring the condition of a0 subject to provide information about treatment efficacy).

The biomarker according to the present application may be used for diagnosing psoriasis by detecting the presence or absence of miRNA and/or detecting the expression level thereof itself, change in expression level or difference in expression levels through quantitative and/or qualitative analysis.

The material for detecting according to the present application is a material that is capable of detecting the presence or absence of miRNA or cRNA or cDNA thereof and/or detecting the expression level thereof itself, change in expression level or difference in expression level, and the composition may further include a reagent that is suitable for detecting the biomarker along with the material for detecting.

In one aspect, the material for detecting includes a probe that specifically binds to the miRNA disclosed in the present application or cRNA or cDNA thereof. The probe refers to a nucleic acid molecule that binds specifically and complementary to a single-stranded RNA or DNA as a template and has a free 3' hydroxyl group that allows reverse transcriptase or DNA polymerase to initiate replication of the template, and this enables the qualitative and/or quantitative measurement of a target by specific binding to the template or target. As described below, it may be used in various methods, and additionally, for the detection of an amplified product, it may be labeled with a chromogenic, luminescent or fluorescent substance, as described below.

A person skilled in the art will be able to select an appropriate probe sequence according to the present application by referring to the sequences of miR-625-3p, miR-4488 or miR-342-3p disclosed in the present application and in miRBase.

In still another aspect, the material for detecting according to the present application is a primer that specifically and complementarily binds to the miRNA disclosed in the present application or cRNA or cDNA thereof, and has a free 3' end hydroxyl group that allows reverse transcriptase or DNA polymerase to initiate the replication of a template. Primers are generally designed to bind to the vicinity of both ends of a site to be amplified in a target nucleic acid in the nucleic acid amplification reaction, and specifically bind to a template (or target) to perform the qualitative and/or quantitative analysis of a template or target material, and they can be used in various methods as described below. In addition, for the detection of an amplified product, it may be labeled with a chromogenic, luminescent or fluorescent substance as described below.

A person skilled in the art will be able to select an appropriate probe sequence according to the present application by referring to the sequences of miR-625-3p, miR-4488 or miR-342-3p disclosed in the present application and in miRBase.

In still another embodiment, the material for detecting according to the present application includes a probe and a primer pair that specifically bind to the miRNA disclosed in the present application or cRNA or cDNA thereof, and in this case, the probe is located between the primer pair.

Probes and/or primers that are capable of detecting miR-625-3p, miR-4488 or miR-342-3p according to the present application may be used in various known methods, and various reagents may also be included in the compositions according to the present application depending on the specific method used.

Such methods include, for example, nucleic acid hybridization, polymerization, amplification methods and hybridization-based ligation, but the present invention is not limited thereto.

Nucleic acid hybridization may be performed in the form of nucleic acids bound to a solid phase support, such as beads, nanoparticles or biochip arrays (microarrays), or by using in-situ hybridization. The miRNA microarray technology enables the analysis of multiple miRNAs simultaneously. Nucleotides that are complementary to miRNAs according to the present application may be spotted on a coated solid support or spotted on a solid support using an in-situ synthesis method. In an exemplary embodiment, miRNA isolated from a biological sample may be detected by the incorporation of a label (e.g., biotin, fluorescent dye) detected by a hybridization enzyme reaction with a complementary sequence on the solid support, for example, a probe. In another exemplary embodiment, miRNA isolated from a biological sample is labeled with a fluorescent material and binds to a corresponding sequence, and as a result, the emitted fluorescence signal indicates the presence of a specific miRNA. For microarray fabrication techniques, for example, reference may be made to Schena et al., 1996, Proc Natl Acad Sci USA.93(20): 10614-9; Schena et al., 1995, Science 270(5235):467-70; and U.S. Pat. Nos. 5,599,695, 5,556,752 or 5,631,734. In this case, the material or reagent for detecting may be provided in the form of being bound to a solid support. The detection reagent may be labeled directly or indirectly in a sandwich form for detection, and reference may be made to the content described below.

The nucleic acid polymerization or amplification method may also be used to detect miRNA according to the present application, and it is particularly suitable for detecting miRNA present in a small amount. Various known nucleic acid amplification or synthesis methods may be used, and for example, it may include reverse transcription reaction, reverse transcription polymerase chain reaction (RT-PCR), real-time RT-PCR, PCR, real-time PCR, quantitative RT-PCR, quantitative PCR, NASBA (Nucleic Acid Sequence-Base Amplification), LCR (Ligase Chain Reaction), multiple ligatable probe amplification, Invader technology (Third Wave), SDA (Strand Displacement Amplification), TMA (Transcription Mediated Amplification), Eberwine RNA amplification and the like, but the present invention is not limited thereto.

In order to amplify a specific target sequence, in the typical PCR method, 3 steps which consist of the denaturation of a template, annealing in which forward and reverse primers bind to the target sequence and elongation with a thermostable polymerase are performed in several cycles, for example, 20 or more times. Alternatively, annealing and elongation may be performed in the same step. Since mature miRNAs are single-stranded, a reverse transcription reaction may be performed first before PCR. The reverse transcription reaction requires the use of primers and reverse transcriptase.

In PCR and quantitative PCR, a set of forward and reverse primers or probes together with the primers may be used. The length of probes and primers is determined according to various factors such as hybridization temperature, the composition of a target sequence, and the complexity of a target sequence. For example, the probe is 7 nucleotides or more, and the length of the primer is about 10 to 35 nucleotides, such as 15, 20, 25, 30 or 35 nucleotides. The forward primer includes at least one sequence that is capable of specifically binding to the biomarker miRNA, and it may further include a non-complementary sequence at the 5' side. The sequence of the reverse primer may be independent of the sequence of the biomarker, and multiple miRNA biomarkers may be amplified with one type of reverse primer, or may include one or more sequences that are specific to the biomarker.

For example, the primer which is capable of amplifying miR-625-3p may be MystiCq^{®} microRNA qPCR Assay Primer hsa-miR-625-3p, but the present invention is not limited thereto. In another aspect, the primers that are capable of amplifying miR-625-3p may be forward: 5'-GCGCAGGACTATAGAACTTTC-3' and reverse: 5'-GGTCCAGTTTTTTTTTTTTTTTGAG-3', but the present invention is not limited thereto.

Amplification products may be analyzed during or after amplification by various methods known in the art. These methods are known in the art and include, for example, gel electrophoresis, real-time PCR analysis, SSCP (single strand conformational polymorphism), RFLP (restriction fragment length polymorphism), CZE (capillary zone electrophoresis), WAVE (HPLC-based nucleic acid analyzing technology) and microchip, but the present invention is not limited thereto.

In one embodiment according to the present application, the real-time quantitative PCR method, that is, RT-PCR is used after reverse transcription reaction, which isolates RNA from a sample, and then, primers, for example, a stem-loop that is capable of forming a stem-loop may be used to synthesize, and then, by using the same as a template, herein, the stem-loop RT-based nucleic acid amplification method using TaqMan-type probes which use forward and reverse primers, or a combination of forward and reverse primers and probes as end-points, or use dyes that bind to nucleic acids such as SYBR in real time, or are labeled with a fluorophore is used. For such a method, reference may be made to, for example, Schmittgen, T.D. et al (2008) Real-time PCR quantification of precursor and mature microRNA. Methods 44, 31.8.; and Chen et al., Nucleic Acids Research, 33(20):e179, 2005.

In addition, the hybridization-based ligation technique may be used for the quantitative analysis of miRNAs. Such methods are known in the art, and include, for example, OLA (oligonucleotide ligation), and for example, a method for separating detectable probes bound to a target nucleic acid sequence from unbound probes, such as the method using HARP-like probes described in U.S. Patent Application Publication No. 2006-0078894, but the present invention is not limited thereto. Another technique using ligation is MLPA (Multiplex Ligation-dependent Probe Amplification) (Schouten et al., Nucleic Acids Research 30:e57 (2002)). The technique combines in such a way that ligation occurs only when a pair of probes binds to the target sequence side by side, and the ligated probe includes a primer binding site such that it can be amplified by PCR.

In the hybridization, amplification, and/or hybridization-based ligation reaction described above, hybridized or amplified miRNA products may be detected through the staining or labeling of a target, the staining or labeling of primers or probes. A known technique in the art may be used for detection, and a person skilled in the art may select an appropriate method in consideration of detection sensitivity and/or target quantity. Depending on the amount of target and/or the sensitivity of the detection method, amplification may not be necessary prior to detection.

In addition, miRNAs may be detected by a direct or indirect method. In the direct method, miRNA is labeled with a detectable label attached thereto, and then bound to a probe linked to a solid support such as a bead, and then, the labeled miRNA is screened for detection. Alternatively, a labeled probe may be used for direct detection, and detection is performed through screening of the labeled probe after specifically binding to the miRNA. In an exemplary embodiment, the amplified miRNA is detected using beads which are conjugated with a probe that is capable of capturing a target nucleic acid. In another exemplary embodiment, the probe may be labeled with a fluorophore. The indirect detection method may also be used. For example, biotinylated probes may be used to detect bound nucleic acids using streptavidin-conjugated dyes. The streptavidin molecule binds to the biotin label of the amplified miRNA, and the bound miRNA is detected by a dye that is conjugated to streptavidin. Dyes that are conjugated to such streptavidin are known in the art, and for example, Phycolink(R) Streptavidin R-Phycoerythrin (PROzyme) may be used.

Labels for detection include, but are not limited to, compounds that are capable of generating or quenching detectable fluorescence, chemiluminescence or bioluminescence signals, such as light-emitting, light-scattering and light-absorbing materials, and for example, reference may be made to Garman A., Non_Radioactive Labeling, Academic Press 1997. Fluorescent materials include, but are not limited to, fluorescein (e.g., US Patent No. 6,020,481), rhodamine (e.g., US Patent No. 6,191,278), benzophenoxazine (e.g., US Patent No. 6,140,500), energy transfer fluorescent dyes including a donor and an acceptor (e.g., US Patent Application Publication No. 10-2016-0022017, US Patent No. 5,945,526) and cyanine (e.g., WO1997-45539), lisamine, phycoerythrin, Cy2, Cy3, Cy3.5, Cy5, Cy5.5, Cy7, FluorX (Amersham), Alexa 350, Alexa 430, AMCA, BODIPY 630/650, BODIPY 650/665, BODIPY-FL, BODIPYR6G,BODIPY-TMR, BODIPY-TRX, Cascade Blue, 6-FAM, Fluorescein Isothiocyanate, HEX, 6-JOE, Oregon Green 488, Oregon Green 500, Oregon Green 514, Pacific Blue, REG, Rhodamine Green, Rhodamine Red, Renographin, ROX, SYPRO, TAMRA, Tetramethylrhodamine and/or Texas Red, and certainly, it includes any fluorescent moiety which is capable of producing other detectable signals. The fluorescent dye includes 6-carboxyfluorescein; 2',4',1,4-tetrachlorofluorescein; and 2',4',5',7',1,4-hexachlorofluorescein, but the present invention is not limited thereto. In an exemplary embodiment, SYBR-Green, 6-carboxy fluorescein ("FAM"), TET, ROX, VICTM or JOE is used as a fluorescent label. In an exemplary embodiment, a probe which is labeled with two fluorescent materials of a reporter fluorescent material and a scavenger fluorescent material is used, and in this case, a fluorescent material that emits a spectrum of distinguishable wavelengths is used as the fluorescent material.

In addition, as the marker, a compound that is capable of improving, stabilizing or influencing nucleic acid binding may be used, and for example, an intercalator including etcidium bromide and SYBR-Green, a minor groove conjugate and a cross-linkable functional group may be used, but the present invention is not limited to, and reference may be made to Blackburn et al., eds. "DNA and RNA Structure" in Nucleic Acids in Chemistry and Biology (1996).

The miRNA quantification may also be performed with reference to the following documents, and for example, reference may be made to miRNA microarrays (Calin, G.A. et al.(2004) Proc Natl Acad Sci USA 101,11755.60.), SYBR-based miRNA RT-qPCR assays (Sharbati-Tehrani et al.(2008) miR-Q: a novel quantitative RT-PCR approach for the expression profiling of small RNA molecules such as miRNAs in a complex sample. BMC Mol Biol 9, 34.), BeadArray (Chen, J. et al. (2008) Highly sensitive and specific microRNA expression profiling using BeadArray technology. Nucleic Acids Res 36, e87.), Invader Assays (Allawi, H.T. et al.(2004) Quantitationof microRNAs using a modified Invader assay. RNA 10, 1153.61.), and Padlock probe-based assays (Jonstrup, S.P. et al.(2006) AmicroRNA detection system based on padlockprobes and rolling circle amplification. RNA 12, 1747.52.).

In addition, commercially available kits based on RT-PCR using primers and probes may be used, and for example, Stem-loop RT based TaqMan MicroRNAAssays (ThermoFisher Scientific, USA) may be used.

Accordingly, the composition according to the present application may include reagents used in any one or more of the methods described above.

The biomarker or composition including the biomarker according to the present application may be advantageously used for the diagnosis of psoriasis, the prediction of recurrence and/or prognosis, and/or the prediction of response after drug treatment.

In another aspect, the present invention relates to the use of a miR-625-3p biomarker for diagnosing the severity of psoriasis. In this case, the present invention may be interpreted as the use of a composition for diagnosing the severity of psoriasis, including a material for detecting a miR-625-3p biomarker, in the diagnosis of the severity of psoriasis.

In still another aspect, the present invention relates to the use of a miR-625-3p biomarker in the preparation of a reagent for diagnosing the severity of psoriasis. In this case, the present invention may be interpreted as the use of a composition for diagnosing the severity of psoriasis, including a material for detecting the miR-625-3p biomarker, in the preparation of a reagent for diagnosing the severity of psoriasis.

In still another aspect, the present invention provides a method for diagnosing the severity of psoriasis, including the step of measuring the expression level of a miR-625-3p biomarker in a subject in need of the diagnosis of severity of psoriasis.

In the above use or diagnostic method, the detailed description of the composition for diagnosing may also be applied to the use and diagnostic method according to the present invention, unless it is interpreted to be contradictory to a person skilled in the art. For example, the miR-625-3p biomarker may be utilized for diagnosing the severity of psoriasis by measuring the expression level thereof using any one or more of a primer, a probe and an antisense nucleotide that specifically binds to the miR-625-3p biomarker.

In still another aspect, the miR-625-3p biomarker may be utilized together with the miR-4488 biomarker and/or the miR-342-3p biomarker to diagnose the severity of psoriasis.

Therefore, the present invention may provide the use of the miR-4488 biomarker and/or the miR-342-3p biomarker for diagnosing the severity of psoriasis together with the miR-625-3p biomarker. In this case, it may be interpreted as the use of a composition for diagnosing the severity of psoriasis, further including a material for detecting a miR-4488 biomarker and/or a miR-342-3p biomarker, in the diagnosis of the severity of psoriasis. Alternatively, the present invention may be interpreted as the use of a composition for diagnosing the severity of psoriasis, including a material for detecting a miR-4488 biomarker and/or a miR-342-3p biomarker, in the preparation of a reagent for diagnosing the severity of psoriasis.

In the present invention, the expression level of the miR-4488 biomarker and/or the miR-342-3p biomarker is measured by using any one or more of a primer, a probe and an antisense nucleotide that specifically binds to each marker, and thus, it may be utilized to diagnose the severity of psoriasis.

In another aspect, the present invention relates to a kit for diagnosing the severity of psoriasis, including the composition.

Reagents that may be included in the kit and detection using the same may refer to the above-mentioned content. In one aspect according to the present disclosure, the kit is used for nucleic acid amplification, and particularly used for amplification using RT-PCR. In this case, the kit may include a primer set and/or a probe, buffer, reverse transcriptase and Taq polymerase that are necessary for the RT-PCR reaction. In another aspect, it may further include a nuclease that is capable of removing single-stranded nucleic acids. Various buffers known in the art may be used, and for example, Tris-HCl, pH 9.0 buffer may be used, but the present invention is not limited thereto. Reverse transcriptase and Taq polymerase are commercially available, and for example, AmpliTaq Gold (Applied Biosystems, USA) which is capable of hot start reaction as a polymerase may be used, and an appropriate concentration of, for example, 1.5 mM to 2.5 mM of MgCl₂ may be included.

The kit according to the present application further includes a positive control, a negative control and instructions for use. The negative control may include a sample that does not include miRNA, and the positive control may include at least one miRNA to be detected.

In still another aspect, the present invention may provide information that is necessary for a method for diagnosing moderate-to-severe psoriasis or for diagnosing or prognosing psoriasis.

Accordingly, the present invention relates to a method for providing information for diagnosing moderate-to-severe psoriasis, including the steps of (a) measuring the expression level of miR-625-3p in a sample isolated from a patient; and (b) comparing the expression level of the miR-625-3p with the expression level of miR-625-3p in a mild psoriasis sample.

In the present invention, the expression level of miR-625-3p may be measured by using reverse transcriptase polymerase reaction, competitive reverse transcriptase polymerase reaction, real-time reverse transcriptase polymerase reaction, RNase protection assay or gene chip, but the present invention is not limited thereto.

In the present invention, the sample may be a tissue, cell, blood or serum sample, but the present invention is not limited thereto.

In the present invention, the sample may include exosomes isolated from tissue, cells, blood or serum, but the present invention is not limited thereto.

In the present invention, when the expression level of miR-625-3p increases by about 3.46 times or more compared to the expression level of miR-625-3p in a mild sample, moderate-severe psoriasis may be diagnosed, but the present invention is not limited thereto.

In the present invention, step (a) may further measure the expression level of miR-4488 and/or miR-342-3p in the sample isolated from the patient.

In addition, step (b) may compare the expression level of the miR-4488 with the expression level of miR-4488 in the mild psoriasis sample and/or compare the expression level of the miR-342-3p with the expression level of miR-342-3p in the mild psoriasis sample.

In the present invention, when the expression level of the miR-4488 increases by about 2.375 times or more compared to the expression level of miR-4488 in the mild psoriasis sample and/or the expression level of the miR-342-3p increases by 1.657 times or more compared to the expression level of miR-342-3p in the mild psoriasis sample, it may be diagnosed as moderate-to-severe psoriasis.

That is, in the present invention, the expression level of miR-4488 and/or miR-342-3p may be additionally verified along with the expression level of miR-625-3p to diagnose moderate to severe psoriasis, but the present invention is not limited thereto.

In the present invention, the expression level of the miR-4488 and/or miR-342-3p may be measured by reverse transcriptase polymerase reaction, competitive reverse transcriptase polymerase reaction, real-time reverse transcriptase polymerase reaction, RNase protection assay or gene chip, but the present invention is not limited thereto.

Specific means (methods) and reagents for detection by which the method according to the present application is implemented may refer to the above description.

In the present application, the sample may be a biological sample, and the term "biological sample" refers to a biologically derived organ, tissue, cell or body fluid.

Examples of biological samples include tissue sections, whole blood, plasma, serum, urine or blood derived leukocytes, red blood cells or platelets, or tissue or cell cultures, but the present invention is not limited thereto. In addition, one or more of the above samples may be mixed and used. Such a biological sample may be directly obtained from a patient who is suspected of having psoriasis immediately before the test by a conventional sample obtaining method, or may be previously separated and stored. In an exemplary embodiment according to the present application, skin tissue or blood samples are used. In the present invention, skin tissue or blood obtained from a subject with or suspected or likely to have psoriasis may be used, but the present invention is not limited thereto.

A subject in the present application includes a mammal that is suspected of having a disease, a mammal suffering from a disease and then treated but suspected of recurrence, and particularly a human.

In the present application, when the expression levels of miR-625-3p, miR-4488 and/or miR-342-3p in the subject (e.g., severely ill patient) are respectively compared with the expression levels of miR-625-3p, miR-4488 and/or miR-3488 in a mild patient sample, for the accuracy of diagnosis, the subject sample and the normal sample may be characterized by comparing the expression levels of the above-mentioned biomarkers after normalization to miR-103-3p, but the present invention is not limited thereto, and it is apparent in the art that miR-103-3p may be normalized to other miRNAs with uniform expression levels in cells.

The composition according to the present application may detect the expression level of the at least one miRNA in a biological sample, compare the same with a control group or reference group, and predict the risk of diagnosis or recurrence or occurrence of psoriasis according to the degree of a decrease or change in the expression level.

In the method according to the present application, the control group is a sample showing differential expression from moderate-to-severe psoriasis disease, and a sample derived from a subject of the same species having mild psoriasis disease is used.

In one aspect according to the present application, when the expression level of the miR-625-3p is compared with the expression level of miR-625-3p of a mild psoriasis sample and the miR-625-3p expression level is increased, for example, by about 10%, about 20%, about 30%, about 40%, about 50%, about 60%, about 70%, about 80%, about 90% or about 100% or more, it may be judged as moderate-to-severe psoriasis or as having an increased or high risk of developing the same.

In still another aspect according to the present application, when the expression levels of miR-4488 and/or miR-342-3p are compared with the expression levels of miR-4488 and/or miR-342-3p in a mild psoriasis sample along with an increased in the expression level of the miR-625-3p and the expression levels of the miR-4488 and/or miR-342-3p are increased, for example, by about 10%, about 20%, about 30%, about 40%, about 50%, about 60%, about 70%, about 80%, about 90% or about 100% or more, it may be judged as moderate-to-severe psoriasis or having an additionally increased or higher risk of developing the same.

The biomarkers according to the present application may be used together with existing markers and/or diagnostic methods. For example, it may be used with PASI and BSA results.

In the method for providing information according to the present invention, the miRNA expression level of mild psoriasis samples may be published in literature or previously measured values, and it may be measured in advance in subjects with mild psoriasis.

### Example

Hereinafter, the present invention will be described in more detail through examples. These examples are only for describing the present invention in more detail, and it will be apparent to those skilled in the art that the scope of the present invention is not limited by these examples according to the gist of the present invention.

### Example 1. Confirmation of exosome-derived miRNA expression patterns according to psoriasis-specific and disease activity differences in psoriasis patients

Experiments on patients in the present invention were conducted in accordance with the Declaration of Helsinki Principles with the approval of the Institutional Review Board of Ajou University Hospital (IRB number: AJIRB-BMR-OBS-15-398), and prior consent was obtained from all test participants.

The diagnosis of psoriasis was performed based on clinical and histological features in skin biopsy. PASI and BSA were assessed on the day of sampling.

Plasma samples from psoriasis patients were collected in serum separator tubes and EDTA-treated tubes, and then immediately centrifuged at 3,000 rpm for 10 minutes at 4°C.

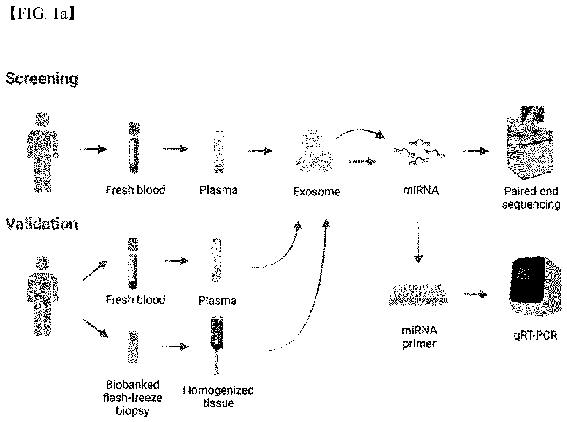

Next generation sequencing (NGS) was performed to select differentially expressed (DE) miRNAs from the blood of 5 patients with PASI > 10 points and 3 patients with PASI < 5 points (FIG. 1a, blue arrow (dotted lines)).

To this end, exosomes were extracted from plasma using the miRCURY^{™} Exosome isolation kit (Qiagen, Hilden, Germany), and total miRNAs were extracted from the exosomes using an RNA isolation kit (miRNeasy Serum/Plasma Kit; Qiagen, Hilden, Germany) according to the manufacturer's instructions.

After cluster amplification of the denatured template according to the manufacturer's instructions, paired-end (150 bp) sequencing was performed by using the Illumina NovaSeq6000 S4 platform for sequencing (Illumina, CA, USA).

The miRNA expression levels were measured by mirdeep2 *(*Nucleic Acids Research. 2012;40(1):37-52) by using the gene annotation database of species with hairpin and mature miRNA sequence information that was extractable from miRBase *(*Nucleic Acids Research. 2014;42(D1):D68-D73). For DEmiRNA analysis, miRNA level count data were generated by using mirdeep2. Based on the calculated read count data, DEmiRNA was identified by using the TCC R package *(*BMC Bioinformatics. 2013;14(1):1-14) for a robust normalization strategy to compare tag count data. Normalization factors were calculated by using the iterative DEGES/edgeR method. Q-values were calculated based on p-values using the p.adjust function of the R package with default parameter settings. DEmiRNAs were identified based on a q-value threshold of less than 0.05 to correct errors due to multiple testing *(*Journal of the Royal Statistical Society: Series B (Methodological). 1995;57(1):289-300).

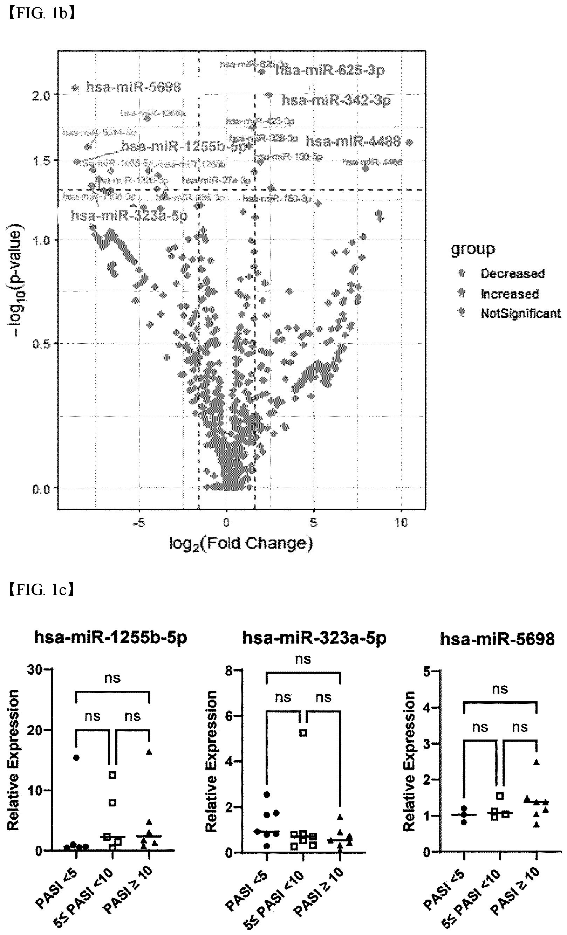

As a result, 19 significant DE miRNAs, that is, 9 upregulated and 10 downregulated miRNAs were identified among a total of 805 detected miRNAs (FIG. 1b). Based on fold change and p-value, miR-625-3p, miR-4488 and miR-342-3p of upregulated miRNAs and miR-5698, miR-1255b-5p and miR323a-5p of downregulated miRNAs were selected, respectively.

In order to verify the possibility of using the previously selected miRNAs as biomarkers for a larger number of patients, according to the PASI score (PASI <5, 5 ≤ PASI <10, PASI ≥10, each group consisted of 14 patients), qRT-PCR was performed using sorted tissue samples (FIG. 1a, red arrows (solid lines)).

Total RNA extraction from plasma samples from psoriasis patients proceeded with the same procedure as described above, skin tissue samples from psoriasis patients were obtained from psoriasis lesions by using a 3 mm disposable biopsy punch, and quick-frozen tissue samples from patients were stored until the time of analysis in a -80°C nitrogen tank at Ajou University Hospital Human Biobank (AUHHB). Skin tissue samples were homogenized in QIAzol Lysis Reagent, and total RNA was extracted by pulverizing lesion tissue using TissueLyser II (Qiagen, Hilden, Germany).

The cDNA was synthesized from the total RNA extracted using miRCURY LNA miRNA PCR Assays (Qiagen) with the miRCURY LNA RT Kit (Qiagen) according to the manufacturer's instructions, and the quantity and quality of the total RNA was evaluated by the spectrophotometric method (Nanodrop^{™} ND-1000, ThermoFisherScientific, Copenhagen, Denmark).

The qRT-PCR was performed by using the QuantStudio 3 Real-Time PCR System (Applied Biosystems, Foster City, CA, USA). The miRNA expression values obtained by qRT-PCR were normalized to miR-103-3p *(*International Journal of Molecular Sciences. 2019;20(18):4353). Afterwards, the results were quantitatively analyzed by using the relative standard curve method (2-ΔΔ).

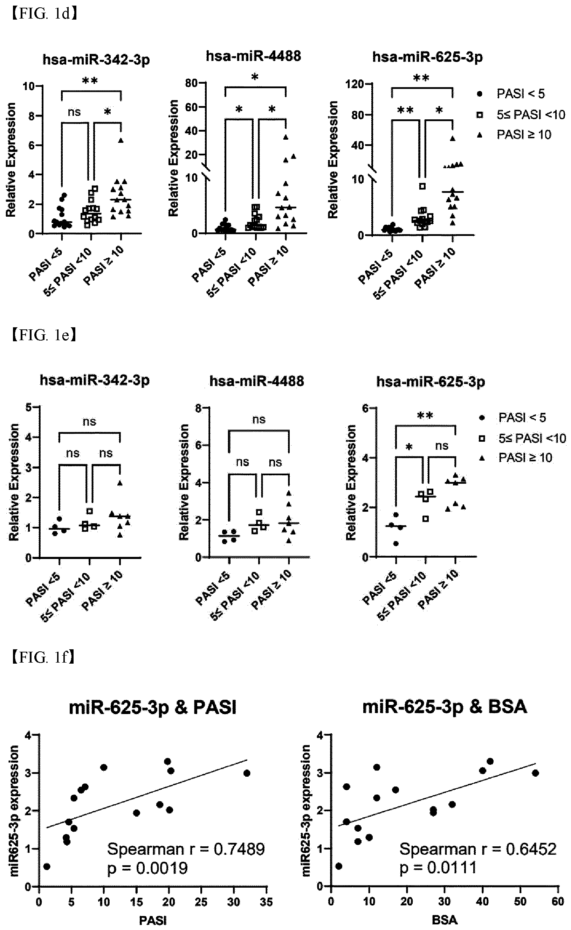

As a result, the selected downregulated miRNAs did not show a significant difference in expression between the groups (Fig. 1c), but all of the selected upregulated miRNAs showed a significant difference in expression between the PASI < 5 group and the PASI ≥ 10 group. In particular, both of miR-4488 and miR-625-3p showed significant expression differences between the PASI < 5 group and the 5≤ PASI <10 group (FIG. 1d).

Meanwhile, in the results using exosomal miRNA isolated from skin tissue, miR-625-3p showed significant differences between the PASI<5 group and the 5≤PAS1<10 group, and the PASI<5 group and the PASI≥10 group (FIG. 1e). In particular, miR-625-3p was a miRNA with a strong association (0.60 ≤ ρ) between PASI and BSA (FIG. 1f, miR-4488 and PASI: Spearman ρ: 0.5862, p = 0.0236, miR-342-3p and PASI: Spearman ρ: 0.3718, p = 0.1719).

### Example 2. Correlation between miR-625-3p and the severity of psoriasis, disease specificity and expression confirmation according to treatment response

Through Spearman correlation analysis, the association between the relative expression levels of miR-625-3p, miR-4488 and miR-342-3p, which were elevatedly expressed in 42 psoriasis patients additionally analyzed in Example 1, and the psoriasis severity index, PASI or BSA, was analyzed.

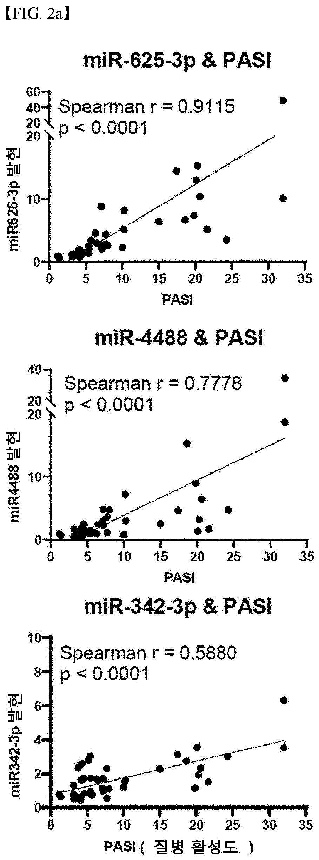

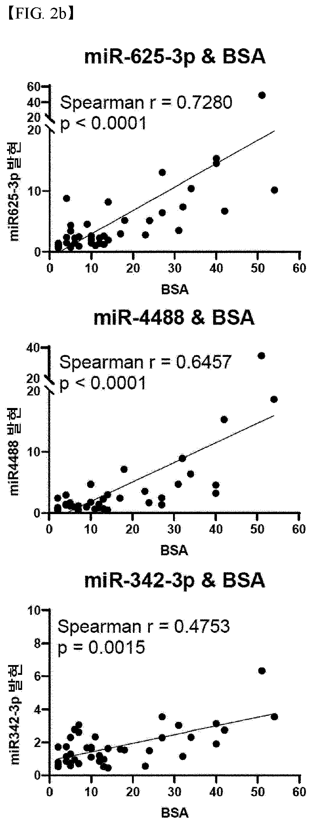

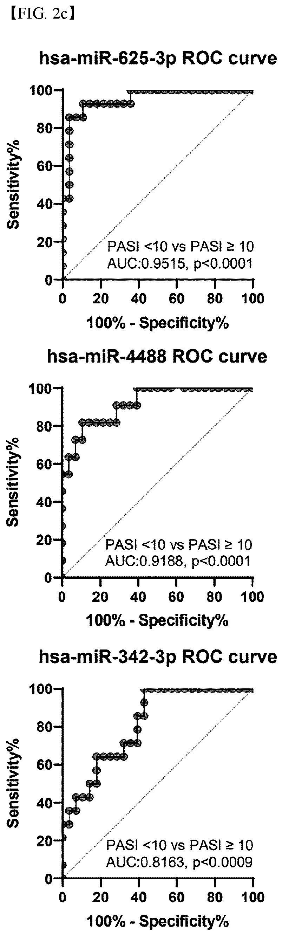

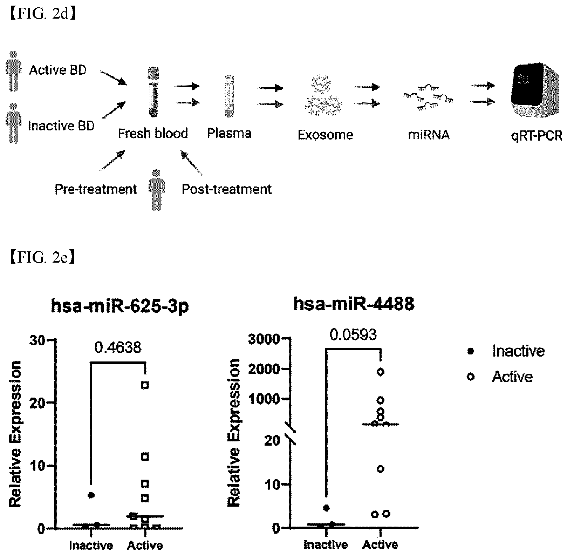

As a result, the 3 types of the selected miRNAs showed a significant correlation (p < 0.0001) between the expression levels and PASI, but miR-625-3p showed an exceptionally high level of correlation (FIG. 2a). Similar results were observed in the correlation with BSA, and miR-625-3p showed the highest correlation coefficient value compared to other miRNAs (FIG. 2b).

Meanwhile, the ROC curve was analyzed to what extent the elevatedly expressed miRNAs could predict the two patient groups of PASI < 10 and PASI ≥ 10.

As a result, miR-4488 was also found to have significant results in the AUC value in the ROC curve, but particularly, miR-625-3p had the highest AUC value with an exceptional value of 0.9515, which confirmed the diagnostic value as a biomarker for determining the severity of psoriasis which distinguished between mild psoriasis (PASI <10) and severe psoriasis (PASI ≥10).

Meanwhile, in order to exclude the possibility that the miRNA is a marker of inflammation that is not specific to psoriasis, circulating exosomal miRNAs from Behçet's disease (BD) patients were additionally analyzed (FIG. 2d, blue arrow (dotted lines)).

To this end, the plasma of 12 patients with Behçet's disease was collected, and after exosomes were extracted, it was determined through qRT-SCR whether the selected miRNA reflects the treatment response based on active/inactive or the presence/absence of treatment response to oral medication. Sample preparation and qRT-PCR were performed in the same manner as in Example 1.

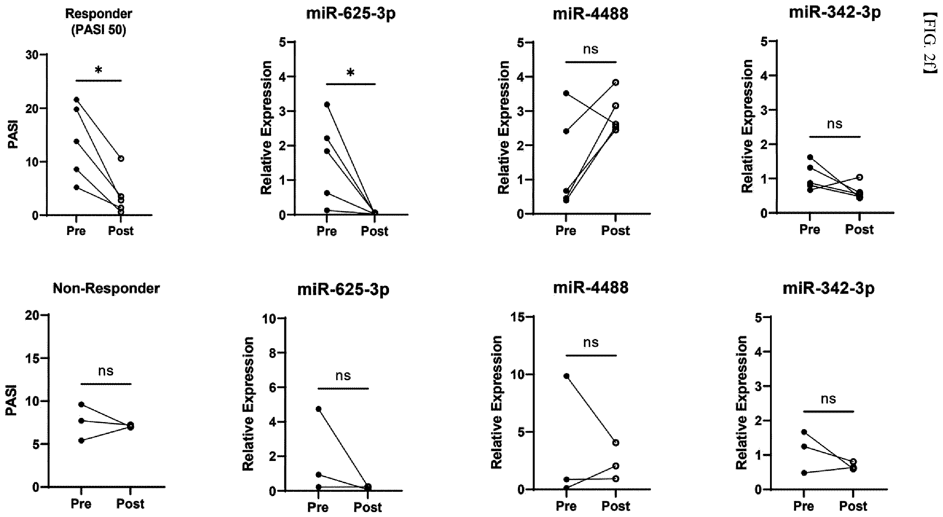

As a result, responders (those who reached PASI 50 after treatment) showed a significant decrease in the expression level of miR-625-3p (FIG. 2f). Therefore, it was found that miR-625-3p is a biomarker that can most accurately indicate psoriasis activity.

### Example 3. Confirmation of source of exosomal miR-625-3p

In-situ hybridization (ISH) was performed on the paraffinized skin tissue of patients to identify the anatomical location of exosomal miR-625-3p in skin tissue.

For ISH, paraffin-embedded sections which were fixed in formalin were cut to a thickness of 6 µm and mounted on slides, and experiments were performed by using miRCURY LNA miRNA Detection Probe (Qiagen) according to the manufacturer's instructions. When it is briefly explained, the sections of patient lesion specimens were deparaffinized and treated with proteinase K for 10 minutes at 37°C. A hybridization mix was prepared by adding LNA detection probes (Qiagen, 5'-DIG- and 3'-DIG-labeled) for hsa-miR-625-3p and hsa-miR-4488 to miRNA ISH buffer (Qiagen). The hybridization mix was applied to the incubated slide and placed in a hybridizer at a temperature ranging from 55°C to 60°C. Afterwards, the slides were washed with 5X SSC, 1X SSC and 0.2X SSC buffers and incubated with 0.2X SSC buffer at 60°C for 1 hour. After washing thoroughly, the probe was detected by incubating the sections with alkaline phosphatase (AP)-conjugated anti-DIG Ab for 1 hour at RT. U6 was used as a positive control for nuclear staining, and Scram was used as a negative control.

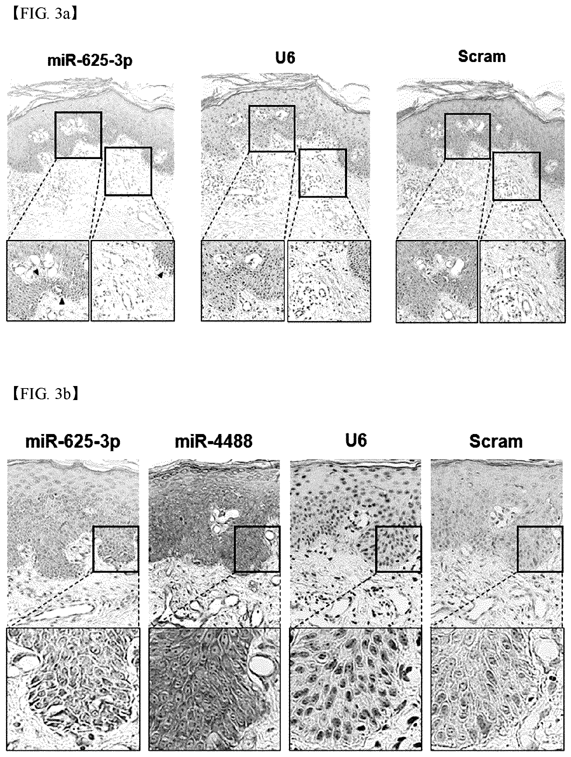

As a result, miR-625-3p was identified in basal keratinocytes, not in infiltrating immune cells (FIG. 3a). Meanwhile, miR-625-3p was observed in the cytoplasm and extracellular matrix of basal keratinocytes, but not in the nucleus (stained with U6) under high-power fields (FIG. 3b).

Accordingly, it was assumed that miR-625-3p is derived from psoriasis basal keratinocytes, and in order to mimic similar conditions, after stimulating HaCaT cells or JurkaT cells with IL-12 (Recombinant Human IL-12 (Cat No. 573002) BioLegend) and IL-23 (Recombinant Human IL-23 (Cat No. 1290-IL) Bio-Techne) (50 ng/mL each), the expression levels of miR-625-3p and miR-4488 were determined through qRT-PCR.

To this end, Immortalized human keratinocytes (HaCaT) cells and T (Jurkat) cells were cultured. The cell lines tested negative for mycoplasma and were cultured in RPMI medium 1640 with L-glutamine supplemented with 10% heat-inactivated FBS. HaCaT cells and Jurkat cells were cultured in a 12-well plate at a concentration of 4 × 10⁴ cells and 5 × 10⁵ cells, respectively, and interleukin (IL)-12 and/or IL-23 were treated in each well plate. These were harvested after 24 hours, and the total RNA in HaCaT and Jurkat cells was isolated with the miRNeasy Micro Kit (Qiagen) according to the manufacturer's instructions, or after extracting exosomes from the culture supernatants of HaCaT and Jurkat cells by the QIAGEN exoRNeasy Midi Kit (Cat. No. 77144), qRT-PCR was performed in the same manner as described in Example 1.

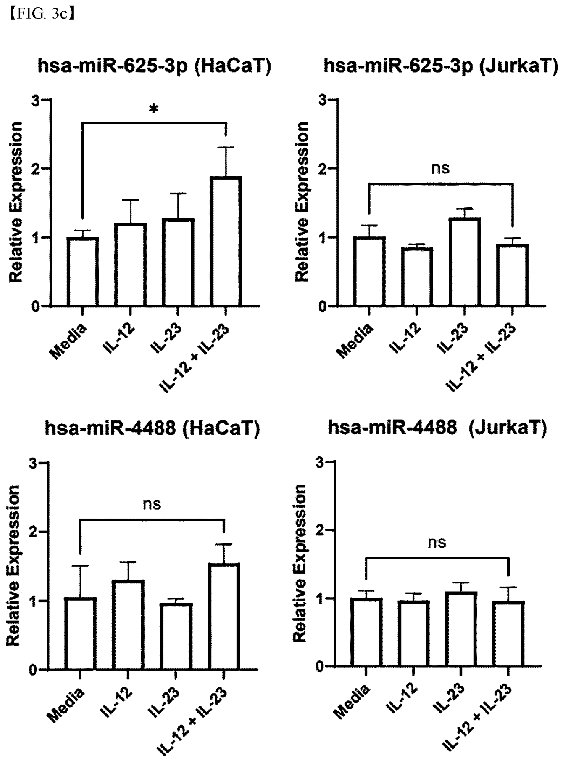

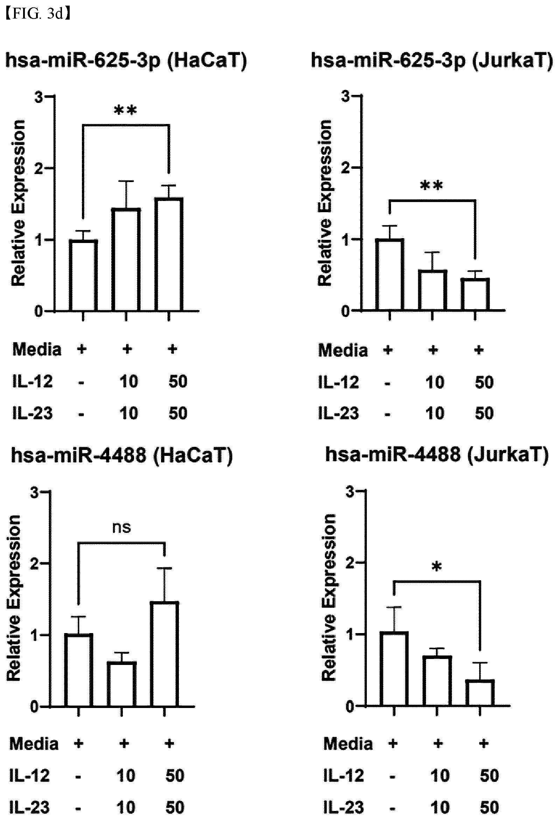

As a result, it was confirmed that the expression of miR-625-3p significantly increased in HaCaT cells that were simultaneously treated with IL-12 and IL-23, but it did not increase in JurkaT cells (FIG. 3c), and it was also found that in the supernatant-derived exosomes of HaCaT cells, the expression of miR-625-3p significantly increased when stimulated with IL-12 and IL-23 (FIG. 3d). Therefore, from the above results, it was found that activated keratinocytes produce miR-625-3p and secrete the same.

### Example 4. Confirmation of correlation between miR-625-3p and IGF-1 signaling system

In order to identify the target of miR-625-3p in psoriasis, the possible role of miR-625-3p in the pathogenesis of psoriasis was investigated by using the miRNA database.

To this end, for miRNA target prediction and functional annotation, 3 online databases of miRDB *(*Nucleic Acids Research. 48(D1):D127-D131 (2020)), TargetScan *(*Genome Biol. 11:R90. (2010)) and miRTarBase *(*Nucleic Acid Res. 8;48(D1):D148-D154 (2020)) were used, respectively. As a result, 294, 2906 and 33 targets were predicted as targets of miR-625-3p in miRDB, TargetScan and mirRTarBase, respectively. The top 100 overlapping genes (6 genes with all 3 overlaps, 94 genes with random 2 overlaps) were selected, the HGNC names were input by using the Enrichr tool *(*BMC Bioinformatics 14, 128 (2013)), and the KEGG pathway and enrichment scores (P-values) for GO terms were obtained. The top 4 (lowest P values) for each category were scored.

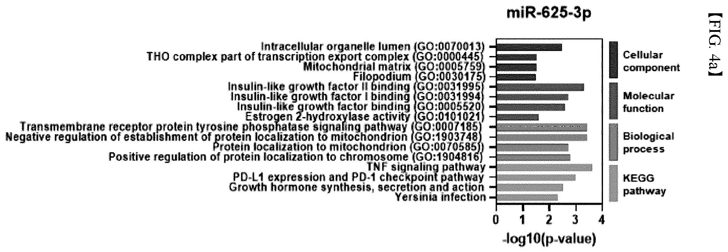

As a result, it was confirmed that miR-625-3p might be correlated with insulin-like growth factor (IGF) (FIG. 4a).

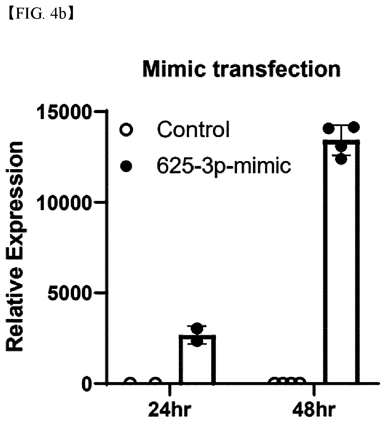

Accordingly, pre-designed *mir*Vana^{™} miRNA Mimic (Negative control, Thermo Fisher Scientific (#4464058)) (or *mir*Vana^{™} miRNA Mimic (has-miR-625-3p, Thermo Fisher Scientific, (#4464066)) was purchased, and after transfection with 50 pmol concentration of miRNA Mimic using Lipofectamine^{®} RNAiMAX reagent according to the manufacturer's instructions (FIG. 4b), qRT-PCR analysis was performed in the same manner as in Example 1 using the following primers in order to confirm the expression levels of various IGF-1 signaling genes.

**[Table 1]**

| Gene name | Forward | Reverse |
|---|---|---|
| IGFIR | GCA CCA TCT TCA AGG GCA ATT TG | AGG AAG GAC AAG GAG ACC AAG G |
| IGFBP1 | AGG CTC TCC ATG TCA CCA AC | CCT GTG CCT TGG CTA AAC TC |
| IGFBP2 | CCT CTA CTC CCT GCA CAT CC | TGC CCG TTC AGA GAC ATC TT |
| IGFBP3 | TGT GGC CAT GAC TGA GGA AA | TGC CAG ACC TTC TTG GGT TT |
| IGFBP4 | ACT CTG CTG GTG CGT CTA CC | TAT CTG GCA GTT GGG GTC TC |
| GAPDH | GAA CGG GAA GCT CAC TGG | GCC TGC TTC ACC ACC TTC T |

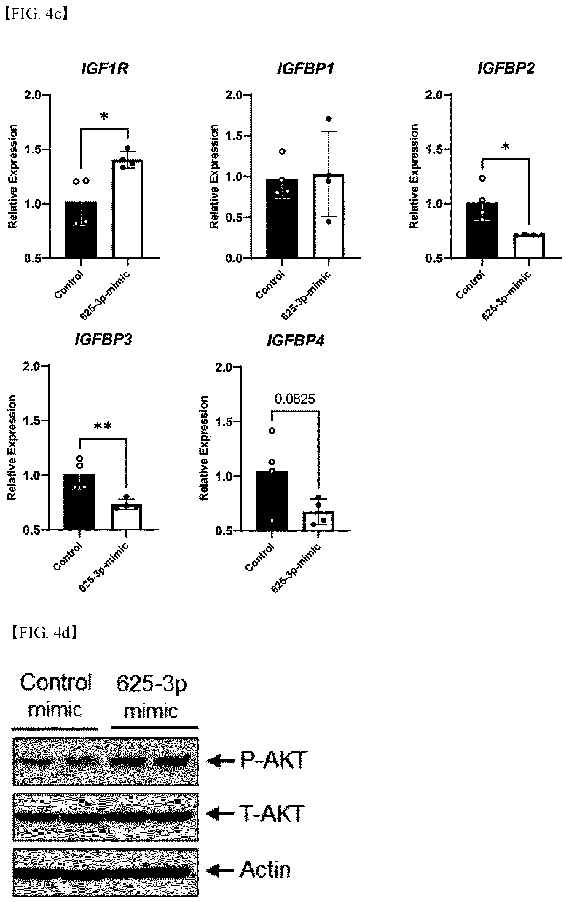

As a result, a significant increase in IGF1R and a decrease in IGFBP2 and IGFBP3 were confirmed (FIG. 4c), and it was found that miR-625-3p could strengthen the IGF-1 signaling pathway.

Meanwhile, if the IGF-1 signal transduction system is strengthened, an increase in p-Akt, which plays a pivotal role in the IGF-1 signal transduction system, can be observed.

Therefore, in order to confirm this, intracellular proteins were isolated from 625-3p mimic-transfected HaCaT cells by using RIPA buffer (including protease inhibitor cocktail). After protein quantification, the proteins were diluted in sodium dodecyl sulfate (SDS) sample buffer and boiled for 5 minutes, and after 20 µg of the protein was electrophoresed on a 10% SDS-polyacrylamide gel, the protein was transferred to a PVDF membrane and blocked with 5% skim milk. After reacting with antibodies to P-Akt (cell signaling; #9271, 1:1000), T-Akt (cell signaling; #9272, 1: 1000), and Actin (Bethyl; A300-491A, 1:5000), respectively, the secondary antibodies were reacted, and the protein bands were determined by using the enhanced chemiluminescence system (Thermo Fisher Scientific). As a result, it was found that p-Akt was actually increased (FIG. 4d).

The increased signaling of IGF-1 can lead to cell proliferation. Since the expression level of the Ki67 gene increases during cell division, the expression level of the Ki67 gene was determined by the qRT-PCR (Ki67-Forward: 5'-GAGAGCTCCCAGCCTAAGGT, Reverse: 5'-CCTGCTTCTCCTTTCCCTTT) method as in Example 1, and cell counting kit analysis was performed by the CCK8 assay using the D-Plus^{™}CCK cell viability assay kit (Dougin; #CCK-3000).

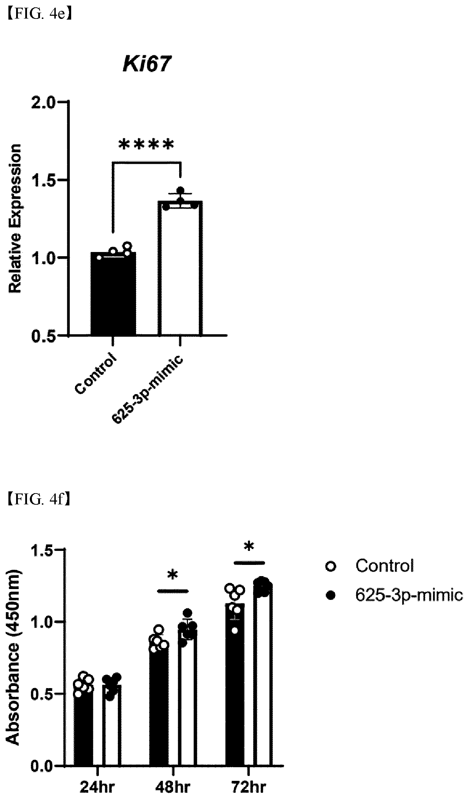

As a result, in the 625-3p mimic-transfected cells, a significant increase in the Ki67 gene (FIG. 4e) and a significant increase in the number of viable cells were observed (FIG. 4f). Therefore, from the above results, it was found that miR-625-3p promotes keratinocyte division through the IGF-1 signaling system.

As described above, specific parts of the content of the present invention have been described in detail, and for one of ordinary skill in the art, it will be clear that these specific descriptions are only preferred exemplary embodiments, and the scope of the present invention is not limited thereby. Accordingly, the substantial scope of the present invention will be defined by the appended claims and their equivalents.

## Claims

1. A composition for diagnosing the severity of psoriasis, comprising a material for detecting a miR-625-3p biomarker.

2. The composition of claim 1, wherein the material for detecting comprises any one or more of a primer, a probe and an antisense nucleotide that specifically binds to the miR-625-3p biomarker.

3. The composition of claim 1, wherein the composition further comprises a material for detecting a miR-4488 biomarker and/or a material for detecting a miR-342-3p biomarker.

4. The composition of claim 3, wherein the material for detecting comprises any one or more of a primer, a probe and an antisense nucleotide that specifically binds to the miR-4488 biomarker and/or the miR-342-3p biomarker.

5. A kit for diagnosing the severity of psoriasis, comprising the composition according to any one of claims 1 to 4.

6. A method for providing information for diagnosing moderate-to-severe psoriasis, comprising the steps of:
(a) measuring the expression level of miR-625-3p in a sample isolated from a patient; and
(b) comparing the expression level of the miR-625-3p with the expression level of miR-625-3p in a mild psoriasis sample.

7. The method of claim 6, wherein the expression level of miR-625-3p is measured by using reverse transcriptase polymerase reaction, competitive reverse transcriptase polymerase reaction, real-time reverse transcriptase polymerase reaction, RNase protection assay or gene chip.

8. The method of claim 6, wherein the sample is a tissue, cell, blood or serum sample.

9. The method of claim 8, wherein the sample comprises exosomes that are isolated from tissue, cells, blood or serum.

10. The method of claim 7, wherein when the expression level of the miR-625-3p increases by 3.46 times or more compared to the expression level of miR-625-3p in a mild psoriasis sample, it is diagnosed as moderate-to-severe psoriasis.

11. The method of claim 7, wherein step (a) additionally measures the expression level of miR-4488 and/or miR-342-3p in the sample isolated from the patient, and
wherein step (b) comprises the step of comparing the expression level of the miR-4488 with the expression level of miR-4488 in the mild psoriasis sample and/or comparing the expression level of the miR-342-3p with the expression level of miR-342-3p in the mild psoriasis sample.

12. The method of claim 11, wherein when the expression level of the miR-4488 increases by 2.375 times or more compared to the expression level of miR-4488 in the mild psoriasis sample and/or the expression level of the miR-342-3p increases by 1.657 times or more compared to the expression level of miR-342-3p in the mild psoriasis sample, it is diagnosed as moderate-to-severe psoriasis.

13. The method of claim 11, wherein the expression level of the miR-4488 and/or miR-342-3p is measured by using reverse transcriptase polymerase reaction, competitive reverse transcriptase polymerase reaction, real-time reverse transcriptase polymerase reaction, RNase protection assay or gene chip.
